# EUROPEAN PATENT SPECIFICATION

(11) **EP 0 444 761 B1**
(45) Date of publication and mention of the grant of the patent: **02.02.1994**
(21) Application number: 91201018.8
(22) Date of filing: 28.05.1987
(51) Int. Cl.: A61M 5/14

(54) **Drug delivery apparatus having chamber with chimney**
Vorrichtung zur Abgabe von Medikamenten mit einer Kammer und einem in diese Kammer ragenden Rohr
Appareil d'administration d'un médicament comportant une chambre avec conduit vertical

(30) Priority: 29.05.1986 US 868826
(43) Date of publication of application: 04.09.1991
(62) Divisional of application: 87903942.8
(73) Proprietor: BAXTER INTERNATIONAL INC., Deerfield, IL 60015 (US)
(72) Inventor: Zdeb, Brian D., Round Lake Park, Illinois 60073 (US); Roeser, Thomas J., Barrington, Illinois 60010 (US); Younkes, William E., Libertyville, Illinois 60048 (US)
(74) Representative: MacGregor, Gordon

(56) References cited:
- EP-A- 0 077 604
- EP-A- 0 146 310
- US-A- 2 612 160
- US-A- 3 208 639
- US-A- 4 552 555

## Description

### Technical Field of the Invention

The present invention is related to the delivery of a beneficial agent to a patient and is more particularly directed to the passive delivery of the beneficial agent to the intravenous system of a patient in a safe and effective manner.

### Background of the Invention

Many drugs are mixed with a diluent before being delivered intravenously to a patient. The diluent may be, for example, a dextrose solution, a saline solution or even water. Many such drugs are supplied in powder form and packaged in glass vials or ampules. Other drugs, such as some used in chemotherapy, are packaged in glass vials or ampules in a liquid state.

Powdered drugs may be reconstituted in a well known manner, utilizing a syringe which is used to inject liquid into the vial for mixing, the syringe eventually withdrawing the mixed solution from the vial. When a drug must be diluted before delivery to a patient the drug is often injected into a container of diluent after it is reconstituted, where the container may be connected to an administration set for delivery to a patient. More specifically, the diluent is often packaged in glass bottles, or flexible plastic containers such as are sold under the names MINI-BAG™ AND VIAFLEX® by Travenol Laboratories, Inc. of Deerfield, Illinois. These containers have administration ports for connection to an administration set which delivers the container contents from the container to the patient. The drug is typically added to the container through an injection site on the container.

Drugs may be packaged separately from the diluent for various reasons. One of the most important reasons is that many drugs do not retain their chemical and physical stability when mixed with a diluent and thus cannot be stored for any substantial period of time. Also, drugs are often packaged separately from the diluent because many firms which manufacture drugs are not engaged in the business of providing medical fluids in containers for intravenous delivery, and vice versa.

Therefore, a doctor, nurse, pharmacist or other medical personnel must mix the drug and diluent. This presents a number of problems. The reconstitution procedure is time consuming and requires aseptic technique. The operator must provide the proper diluent and a syringe before beginning. Often the powdered drug is "caked" at the bottom of the vial. Thus, when liquid is injected into the vial from a syringe the surface area of contact between the liquid and the powdered drug may be quite small initially, thus making the mixing procedure even more time consuming. Because of the limited vial volume, the increasing drug concentration in the diluent makes it harder to finish the reconstitution process. The operator may attempt to solve this by repeatedly injecting solution into the vial, mixing and withdrawing the solution but this makes necessary additional injections and movement of the syringe which increase the likelihood of contamination. Also, it is sometimes difficult to get all of the drug and/or liquid out of the vial, thus increasing the time required to perform the reconstitution procedure.

The reconstitution procedure should be performed under preferably sterile conditions. In addition to such a requirement making the operator justifiably more cautious and consuming more time, sterile conditions are often hard to maintain. In some instances, a laminar flow hood may be required under which the reconstitution procedure is performed.

Some drugs, such as some chemotherapy drugs, are toxic. Exposure of the operator to the drugs during reconstitution may be dangerous, especially if the operator works with such drugs on a daily basis and is repeatedly exposed to them.

A further problem is that the reconstitution procedure provides a source of confusion as to which container contains which drug. The diluent container should be marked with the drug with which it has been injected and the name of the patient to whom it should be delivered.

After a drug is reconstituted and withdrawn into a syringe barrel, the drug may in some instances be injected immediately into the intravenous system of a patient. More typically however, the reconstituted drug is injected from the syringe into a larger container of solution as discussed above, for connection to an intravenous administration set. This is because often the drug reconstituted In the syringe is still at a concentration so high as to cause local toxicity In the veins of a patient near the injection site where the needle pierces the skin. This may create severe vein irritation which may be medically harmful. Additionally, even though the proper dose of medication is in the syringe, immediate injection into the patient's blood stream may create a condition of systemic toxicity wherein the level of drug concentration in the patient's entire blood stream is dangerously high. Yet another reason for not making the injection from the syringe directly into the patient is that it creates an additional injection site into the patient, which may be painful for the patient and provides another opportunity for infection.

For these reasons, the reconstituted drug is more typically injected into a diluent container.

A patient may typically be administered a dextrose or saline solution from a large volume parenteral container, for example, such as a one liter container, delivered through an administration set such as a CONTINU-FLO® administration set sold by Travenol Laboratories. If the reconstituted drug were injected into the large volume parenteral container, delivery of the drug would usually be delivered over too long a time period. Often, these large volume fluids are delivered at very slow flow rates.

More typically, the reconstituted drug is injected into a small volume parenteral container, such as a fifty milliliter container sold by Travenol Laboratories. This MINIBAG™ container is hung at a higher elevation than the large volume parenteral container and is connected by a secondary administration set to an injection site on the primary administration set. Because it is maintained at a higher elevation, the reconstituted drug in the small volume container is delivered, after which fluid from the large volume container begins to flow once more. By utilizing a small volume container connected to an administration set for delivery of the drug or other beneficial agent instead of a direct syringe injection, the drug is delivered over a preferred time period that tends to minimize negative side effects.

A closed reconstitution delivery system is disclosed in U.S. Patent Nos. 4,410,321; 4,411,662; 4,432,755; and 4,458,733, all assigned to Baxter Travenol Laboratories Inc., the assignee of the present invention. As shown therein, a container includes a drug and a diluent in separate compartments which are reconstituted in a closed system before the drug is delivered to the patient. Typically, the container is connected to an administration set which is connected at its other end to the primary administration set, such as with the small volume parenteral container described above. The container shown in these patents solves many of the problems associated with syringe reconstitution. The product does however necessitate a series of reconstitution steps which must be performed by the nurse or other operator prior to delivering the fluid from the container.

Delivery of a drug or other beneficial agent in a manner not requiring reconstitution steps by an operator is shown in U.S. Patent Nos. 4,424,056; 4,432,756; 4,439,183; 4,474,574; 4,479,793; 4,419,794; 4,525,162, and 4,548,599 and Canadian Patent No. 1,173,795, assigned to Alza Corporation of Palo Alto, California. As disclosed in those patents, a parenteral delivery system is disclosed which has a formulation chamber therein for administering a beneficial agent such as a drug. The system is advantageous in that it provides for reconstitution of the drug by fluid flowing from a large volume parenteral container for example, through the administration set containing the formulation chamber with the drug therein. The system intends to eliminate the need for the time consuming reconstitution procedure described above and appears to eliminate the problems associated with the reconstitution procedure.

Another passive reconstitution system is disclosed in European Patent Application No. 0059694 to Aktiebolaget Hassle of Sweden.

Still another device for delivering a drug "in-line", i.e., in the administration set, is disclosed in U.S. Patent No.4,534,757 assigned to Alza Corporation. The device holds the drug and includes a section through which the liquid passes in a direction substantially opposite to the general direction in which liquid flows to the patient.

Yet another system which attempts to provide for drug reconstitution in-line without manual reconstitution by a nurse or other operator is shown in U.S. Patent No. 4,465,471, assigned to Eli Lilly and Co. of Indianapolis, Indiana. That patent discloses constructions for a receptacle in the administration set itself. A separate cartridge containing the drug to be reconstituted and delivered to the patient is plugged into the receptacle. As liquid enters the cartridge for reconstitution of the drug and subsequent delivery out of the cartridge and receptacle and into the patient, some or most fluid continues to flow through the administration set, bypassing the cartridge entirely.

European Patent Application Publication No. 0146310 to Eli Lilly and Co. is directed to a system for drug reconstitution including an intravenous administration set and a drug vial and utilizes the vial vacuum to reconstitute the drug.

U.S. Patent No. 4,534,758 to Akers et al. discloses a relatively complex drug delivery apparatus with various valves. When liquid from a container is delivered to the drug vial, the vial is to be agitated for a time sufficient to suspend the previously dry medicine.

U.S. Patent No. 4,581,014 to Millerd et al. assigned to Ivac Corporation of San Diego, California discloses a selector valve for delivering a previously reconstituted drug from a drug vial through an intravenous administration set to a patient.

All the publications described above are directed to solutions to the time consuming reconstitution procedure and/or its associated problems, such as delivery of the solution to a patient. In most of the offered solutions, delivery of the drug is intended to be passive, i.e., once the drug is placed into the administration set, manual reconstitution steps are not required.

Still another common feature of many of the attempted solutions disclosed in these publications is that delivery of the drug is intended to be able to be made in a manner which is essentially independent of the fluid flow rate through the administration set and into the patient. Stated differently, some of the systems are designed to deliver a certain dosage of drug in a preselected time period, within a broad range of fluid flow rates. Delivery of a drug independent of flow rate is desirable because it ensures that the necessary dosage will be delivered within a therapeutically acceptable time period, which may be typically about twenty to thirty minutes, although this time period may vary depending upon the drug and dosage.

By making delivery of the drug or other beneficial agent independent of the flow rate, the system ensures that the drug will not be delivered too quickly should the flow rate be set too high by the nurse or other operator, thereby preventing the problem of systemic toxicity discussed above.

Some of the documents, such as U.S. Patent Nos. 4,424,056; 4,479,793; and 4,479,794, are also directed to systems having a beneficial agent placed "in-line" in an administration set for mixing of the agent and delivery to a patient, wherein the delivery of the agent may be made in a given volume of fluid. Also, a valve controlling fluid flow may be manually operated to deliver the agent in a manner which can be made dependent upon fluid flow.

It would be desirable to provide a cartridge containing a beneficial agent wherein the cartridge design assures a proper fluid flow path for delivery of the proper amount and concentration of drug to the patient.

The pre-characterising part of Claim 1 is based on the disclosure of EP-A-0146310, referred to above.

The distinguishing features of the invention are set out in the characterising part of Claim 1.

The beneficial agent is contained in a hollow open-ended member, or chimney, extending within a chamber from closure means, penetrable by hollow cannulas. The agent may be retained within the chimney by means of a liquid pervious barrier such as a particulate matter barrier. The particulate matter barrier in the preferred embodiment includes a screen having a nominal pore size of no greater than about 20 microns and most preferably about 5 to 10 microns. As with the previous embodiment, the closure means is adapted to be pierced both at a point in alignment with the interior of the chimney and at a point in alignment with the area of the inner face of the closure means, external of the chimney.

In the preferred embodiment the cartridge includes a flow connector adapted for mounting to the chamber either during or subsequent to manufacture. The flow connector includes a base and two hollow cannulas, pointed at each end, mounted within the base and extending in a direction substantially parallel to the chimney. In this embodiment the first cannula pierces the closure means in alignment with the chimney such that the first cannula is disposed within the chimney. The second cannula pierces the closure means external to the chimney. when the cartridge is mounted upon the receptacle in the administration set, liquid flowing through the receptacle enters the first cannula and subsequently enters the chimney where the beneficial agent is stored. The liquid level rises within the chimney until it overflows into the remainder of the cartridge chamber. Liquid rises within the chamber until it reaches the top of the second cannula, whereupon liquid, with the agent mixed therein flows downstream to the patient.

### Description of the Drawings

Fig 1 is a perspective view of an administration set including the air flask and the receptacle;
Fig 2 is an enlarged, fragmentary, cross-sectional view of the receptacle illustrated in Figure 1;
Fig 3 is a fragmentary, enlarged, cross-sectional view of the air flask illustrated in Fig 1;
Fig 4 is a longitudinal cross-sectional view of a cartridge as being inserted into a receptacle having a piston-like injection site;
Fig 5 is a fragmentary cross-sectional view of the cartridge and receptacle of Fig 4 with the injection site having been moved to its stressed position by full engagement of the cartridge and receptacle;
Fig 6 is a longitudinal cross-sectional view of a cartridge according to the present invention, with a flow connector;
Fig 7 is a fragmentary view of the cartridge and connector of Fig 6, showing cannulas of the connector having penetrated the cartridge; and
Fig 8 is a longitudinal cross-sectional view of the cartridge and connector of Fig 7, showing the connector connecting the cartridge to a receptacle.

### Detailed Description

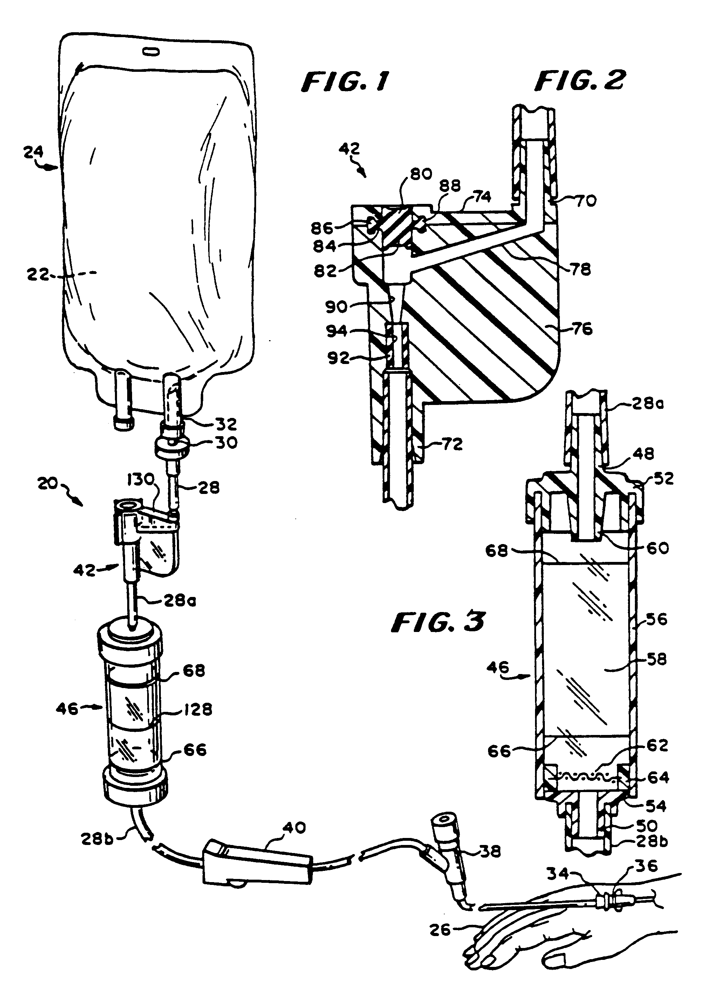

Referring to Fig. 1, there is illustrated an administration set 20 for the delivery of a medical liquid, stored within a medical liquid source such as large volume parenteral container 24, to a patient 26. The administration set 20 includes a fluid conduit 28 made for example of flexible polyvinylchloride tubing. Upstream connection means such as a standard intravenous administration set spike 30 is mounted at the upstream end of the fluid conduit 28. The spike is adapted for piercing the membrane of the container administration port 32.

The fluid conduit 28 includes downstream connection means such as a Luer taper 34 mounted at the downstream end of the fluid conduit 28. The Luer taper 34 may be connected in accordance with standard technique to a venous catheter 36.

The administration set 20 may further include a standard pierceable injection site 38 for injecting a medical liquid by means of a needle through the injection site 38. The administration set 20 may further include flow rate control means such as a standard roller clamp 40 mounted about the flow conduit 28.

The administration set 20 further includes a receptacle 42 shown in greater detail in Fig. 2. The receptacle 42 is mounted along the fluid conduit and is adapted for receiving a separate cartridge 208 containing beneficial agent. When the cartridge is mounted upon the receptacle, at least some, and preferably all liquid from the medical liquid source container 24 that flows through the fluid conduit 28 into the receptacle 42 also flows through the cartridge 208 before passing downstream out of the receptacle to the patient.

Downstream of the receptacle 42 is an air chamber 46, illustrated in Figs. 1 and 3. As will be explained in greater detail below, the air flask 46 permits automatic priming of the cartridge 208 upon mounting of the cartridge on the receptacle 42 of the administration set 20. The air flask 46 absorbs the air disposed within the cartridge 208 and prevents that air from passing downstream to the patient.

Referring to Fig. 3, the air flask 46 includes an inlet 48 mounted to and receiving fluid from upstream fluid conduit 28a. The air flask 46 includes an outlet 50 mounted to and transferring liquid to downstream fluid conduit 28b. The inlet and outlet may be mounted to the fluid conduit 28 by means of interference fit, solvent bonding etc. The air flask 46 is mounted downstream of the receptacle 42.

In the preferred embodiment the air flask 42 includes inlet and outlet end caps 52, 54 respectively between which is mounted a cylindrical sidewall 56 of preferably optically transparent, flexible material such as polyvinyl chloride . The sidewall 56 and the end caps 52, 54 define an air chamber 58 having a cross-sectional diameter that is greater than the internal diameter of the fluid conduit 28, so that liquid entering the air chamber 58 from the drop forming orifice 60 adjacent the inlet 48 falls toward the outlet 50. The air flask 46 thus provides a collection reservoir for air within the administration set 20.

The air flask 46 further includes particulate matter barrier means such as a particulate matter screen 62 mounted within a plastic ring 64 near the outlet 50. The particulate matter barrier may in fact be a sterilizing filter having a nominal pore size of about 0.2 micron. The nominal pore size may be much larger, such as a gross particulate matter barrier having a nominal pore size of about 20 microns. In the preferred embodiment the nominal pore size is about 10 microns. The screen may be a nylon mesh material such as supplied by Filter Tek of Hebron, Illinois. The particulate matter barrier 62 is mounted transverse to the fluid path such that all liquid passing through the air flask 46 must pass through the particulate matter barrier 62.

The particulate matter barrier 62 need not be disposed within the air flask 46 but the barrier should be mounted downstream of the receptacle 42 so that all liquid that exits the inserted cartridge 208 will pass through the particulate matter barrier. Also, it is possible to construct the receptacle 42, the air flask 46 and the particulate matter barrier 62 as a single unit rather than as separated by upstream fluid conduit 28a for example.

In the preferred embodiment, the air flask 46 includes a minimum liquid level indicator 66 and a maximum liquid level indicator 68 which may for example comprise lines around the periphery of the air flask 46. The liquid level in the air flask 46 should preferably be somewhere between the minimum and maximum liquid level indicators 66, 68 immediately before insertion of the cartridge 208 within the receptacle 42.

The improved receptacle 42 includes a receptacle inlet 70 and a receptacle outlet 72 connected to the fluid conduit 28. The air flask 46 is disposed downstream of the receptacle outlet 72.

The receptacle 42 includes upper and lower fitments 74, 76 respectively. The upper fitment 74 includes the inlet. The lower fitment 76 includes the outlet 72 and a fluid receiving segment 78 having an upstream end in fluid communication with the inlet 70 and a downstream end in fluid communication with the outlet 72.

Pierceable situs 80 is mounted within the receptacle 42, trapped between the upper and lower fitments 74, 76. The pierceable situs 80 includes a pierceable main body portion 82 and a ring-like extension 84 extending about the periphery of the main body portion 82. The ring-like extension 84 further includes an enlarged outer periphery 86.

Together, the upper and lower fitments 74, 76 define an annular channel 88 substantially corresponding to and receiving the ring-like extension 84 including the enlarged periphery 86 thereof, such that the upper and lower fitments trap the pierceable situs 80 therebetween in secure fashion. The situs 80 may not be removed without destruction of the receptacle 42. The upper and lower fitments 74, 76 may be bonded together by adhesive, ultrasonic sealing etc. It is important that the situs be securely maintained within the receptacle because a plurality of cartridges 208, each having two piercing cannulas, may be repeatedly inserted and withdrawn from the situs during the useful life of the receptacle 42 and administration set 20. The fluid receiving segment 78 includes a tapered portion 90 below and generally coaxial with the pierceable situs 80. The tapered portion 90 serves as a needle guide into the resilient bushing 92.

The resilient bushing is preferably made of an elastomer such as polyisoprene. The resilient bushing 92 defines a narrow through-bore 94. The resilient bushing is juxtaposed relative to the pierceable situs 80 so that the through-bore 94 is substantially coaxial with the tapered portion 90.

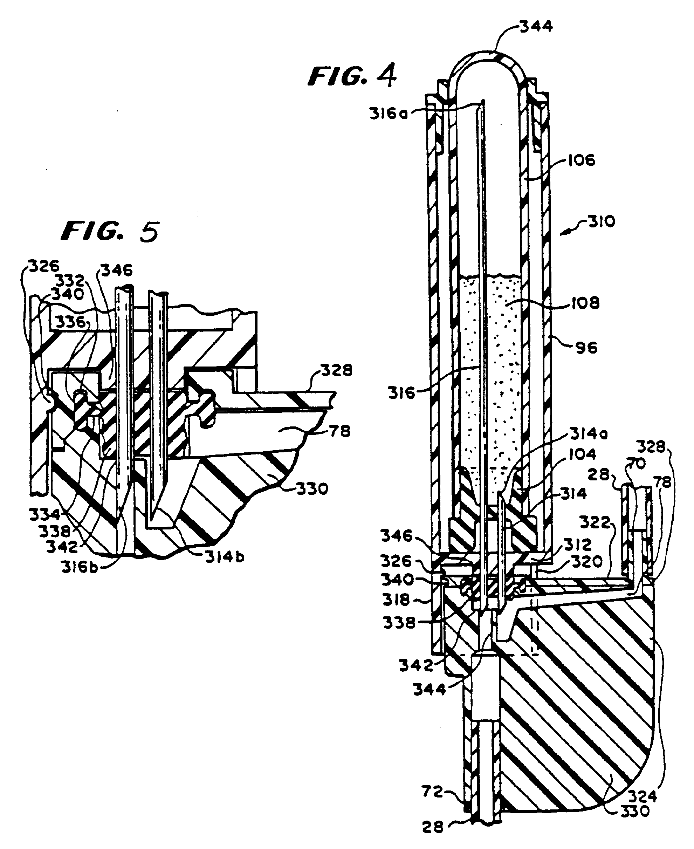

Referring now to Figures 4 and 5, there is illustrated a cartridge 310 (which is not in accordance with the invention), including a rigid cylinder 96 in which is slidably disposed a tubular chamber 106 having beneficial agent 108 therein. A base plate 312 extends across the cylinder 96. Disposed within the base plate 312 are first and second hollow cannulas 314, 316 respectively, each including a first pointed hollow end 314a, 316a respectively facing the tubular chamber 106. The tubular chamber 106 of the cartridge 310 slides from a first position out of engagement with the first and second cannulas to a second position illustrated in Figure 4 where the first ends 314a, 316a of the first and second cannulas 314, 316 have pierced the rubber stopper 104 of the tubular chamber 106.

The cartridge 310 includes a keyway wall 318 extending from the side of the base plate 312 opposite the tubular chamber 106. A keyway slot 320 is defined within the keyway wall 318 for fitting about the bridge 322 of a receptacle 324. The keyway wall 318 includes one or more internal projections 326.

The second pointed hollow ends 314b, 316b of the first and second hollow cannulas 314, 316 respectively may extend the same distance from the base plate 312.

The receptacle 324 includes a receptacle inlet 70 and a receptacle outlet 72 connected to a fluid conduit 28 of an administration set such as the administration set 20. The receptacle 324 includes a fluid receiving segment 78 having an upstream end in flow communication with the inlet 70 and a downstream end in fluid communication with the outlet 72.

The receptacle 324 does not include a bushing such as the bushing 92 in the receptacle 42; however, as will be described below in greater detail, once the cartridge 310 and the receptacle 324 are fully engaged, all liquid flowing through the receptacle must first pass through the tubular chamber 106.

The receptacle 324 includes upper and lower fitments 328, 330 defining an annular channel 332 substantially corresponding to and receiving the ring-like extension 334, including the enlarged periphery 336 thereof, of a pierceable, piston-like injection site 338 made of a resilient pierceable material such as polyisoprene. One or more detents 340 are provided about the exterior of the receptacle 324 for engagement with the internal projections 326 on the keyway wall 318.

An outflow seal 342 may be molded within the lower fitment 330 of the same relatively rigid plastic material as the remainder of the lower fitment 330. The outflow seal 342 defines an outflow channel 344 of larger diameter than the second hollow cannula 316 of the cartridge 310.

In operation, the nurse or other operator pushes down on the top 344 of the tubular chamber 106, sliding it from the first position to the second position illustrated in Fig. 4 where the stopper 104 abuts the base plate 312. The operator then mounts the cartridge 310 about the receptacle 324 in the only manner permitted by the keyway wall 318, keyway slot 320 and bridge 322. As illustrated in Fig. 4, the cannulas 314, 316 both pierce the situs 338. However, as illustrated in Fig. 4, the cartridge 310 is not fully mounted about the receptacle 324. In Fig. 21, the situs 338 is still in its normal position. Liquid flowing into the inlet 70 may flow through the outlet 72 by passing around the outflow seal 342, without entering the chamber 106.

To completely engage the cartridge and the receptacle, the nurse or other operator pushes down further on the rigid cylinder 96 to reach the fully engaged position illustrated in Fig. 5. By exerting downward pressure on the cartridge 310 relative to the receptacle 324, a central raised portion 346 pushes down on the situs 338 causing it to shift downwardly from its normal position illustrated in Fig. 4 to its second, stressed position illustrated in Fig. 22. The situs moves in a direction substantially mutually orthogonal to the ring-like extension of the situs. In the stressed position shown in Fig. 5, the situs 338 seals about the outflow seal 342 of the receptacle. The stressed position of the injection site 338 is maintained by the interfitment of the now-engaged projections 326 and detents 340.

Liquid now flowing into the inlet 70 and fluid receiving segment 78 is necessarily diverted into the first hollow cannula 314 through end 314b and into the chamber 106 containing the beneficial agent 108. The pressure upon the situs 338 causes an effective liquid seal between the situs 338 and the outflow seal 342. Liquid exits the tubular chamber 106 through the second cannula 316 and subsequently flows out of the receptacle through the outlet 72, downstream to the patient.

The cartridge 310 and receptacle 324 combination eliminate the need for the manufacture and assembly of the bushing to create a single flow path once the cartridge is engaged about the receptacle. After the beneficial agent has been delivered to the patient, the operator may remove the cartridge, whereupon the situs 338 returns to its normal position illustrated in Fig 4, so that liquid may flow directly through the receptacle. Subsequent cartridges 310 may be secured through the receptacle 324, at which time the situs is once more forced into the stressed position shown in Fig 5.

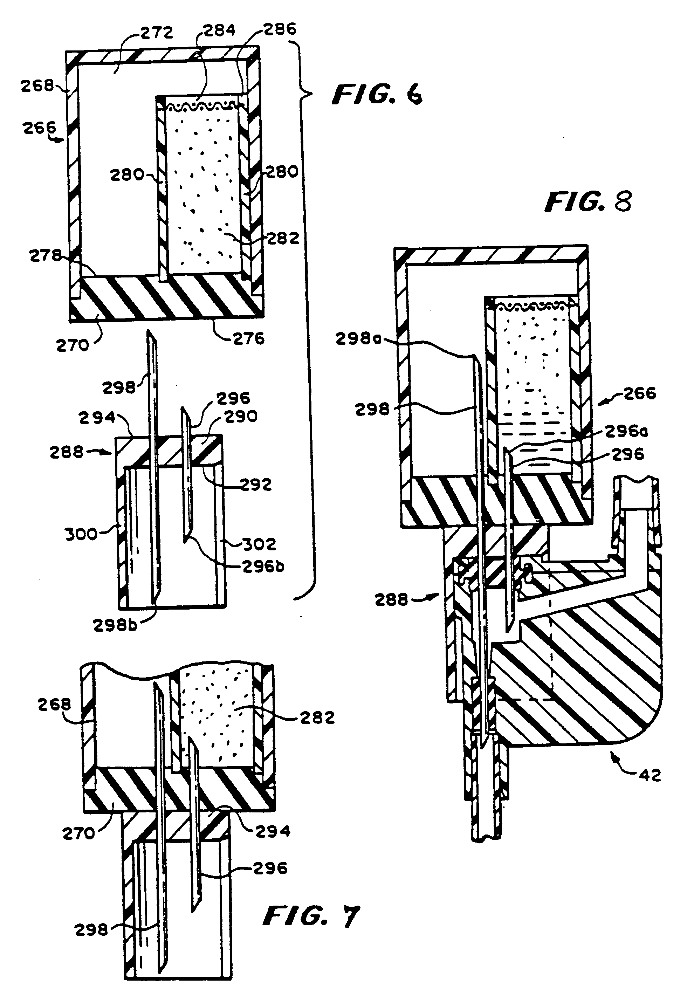

Referring to Figs 6 to 8, there is disclosed a cartridge 266 according to the invention including a wall 268 relatively impermeable to vapor and air and a pierceable closure means such as pierceable stopper 270 which together define a chamber 272. The pierceable stopper 270 includes an outer face 276, and inner face 278 facing the chamber 272.

A chimney 280 extends from the inner face 278 in a direction substantially parallel to the length of the cartridge or, stated differently, in a direction substantially perpendicular to the outer face 276. A beneficial agent 282 is stored within the chamber within the chimney 280 itself. A liquid pervious barrier 284 such as a particulate matter barrier having a nominal pore size no greater than about 20 microns, such as a nylon mesh screen, is mounted at the top 286 of the chimney 280. The liquid pervious barrier 284 retains the beneficial agent 282 within the chimney 280 until such time as the cartridge 266 is plugged into the adapter 42.

The cartridge 266 in the preferred embodiment also includes a flow connector 288 having a base 290. The base 290 includes a chamber distant side 292 and a chamber facing side 294. Mounted within the base 290 are first and second cannulas 296, 298 respectively. An extension wall 300 extends from the chamber distant side 292 of the base 290 and defines a slot 302 enabling mounting of the cartridge 266 upon a receptacle 42.

The first pointed end 296a of the first cannula 296 extends from the base 290 a shorter distance than the first end 298a of the second hollow cannula 298. Similarly, the second pointed end 296b of the first hollow cannula extends from the chamber distant side of the base 290 a shorter distance than the second hollow end 298b of the second cannula 298, for use with the cartridge 44 described above. The disposition of the second hollow cannula ends 296b, 298b may be changed for use with a receptacle such as the receptacle 324 illustrated in Fig 4.

In use, the operator urges the first and second cannulas 296, 298 through the pierceable stopper 270, until the chamber facing side 294 of the base abuts the stopper 270, as illustrated in Fig 7. The first cannula 296 is disposed within the chimney 280. Because the beneficial agent is retained within the chimney, an upward flow path of liquid mixing with beneficial agent is created within the chimney itself.

Eventually liquid reaches the liquid pervious barrier 284 and flows down the outside wall of the chimney 280. The liquid, with the beneficial agent therein, collects within the chamber 272 outside the chimney 280. The liquid level rises until it reaches the level of the first end 298a of the second cannula 298, at which time liquid flows into the second cannula 298 and downstream to the patient. The mounting of the cartridge 266, including the flow connector 288 about a receptacle 42 is illustrated in Fig 8.

## Claims

1. A cartridge (266), for introducing a beneficial agent into a fluid conduit, comprising a wall defining an elongate chamber (268) containing a beneficial agent (282) and pierceable closure means (270) closing an end of the chamber, the closure means (270) having an outer face (276) and an opposed inner face (278) facing the chamber, the closure means being penetrable by the penetrating means to provide for fluid flow into the chamber and out of the chamber, characterised in that the closure means (270) is provided with a hollow open-ended member (280) which extends from said inner face (278) into the chamber in a direction substantially parallel to the length of the chamber, in that the beneficial agent is contained in the hollow open-ended member (280), and in that the closure means (270) is penetrable both at a first location to permit fluid flow through the closure means into the member (280) and at a second location spaced from the member to permit fluid flow out of the chamber (268).

2. A cartridge according to Claim 1, wherein the beneficial agent (282) is retained within the hollow open-ended member (280) by means of a liquid pervious barrier (284).

3. A cartridge according to Claim 2, wherein said barrier (284) comprises a particulate matter barrier.

4. A cartridge according to Claim 3, wherein said barrier (284) has a nominal pore size no larger than 20 microns.

5. A cartridge according to Claim 4, wherein said barrier (284) has a nominal pore size no larger than 10 microns.

6. A parenteral administration set including a cartridge (266) according to any preceding claim, the set also comprising an inlet end (30), an outlet end (34) and a fluid flow path between said ends, the path including a portion provided with penetrating means (296, 298) including a fluid inlet (296b) in communication with said inlet end (30) and a fluid outlet (298b) in communication with said outlet end (34), the closure means (270) being penetrable by the penetrating means to place the fluid inlet (296b) in communication with said hollow open-ended member (280) and the fluid outlet (298b) in communication with the chamber (272) adjacent to the hollow open-ended member (280).

7. The administration set in accordance with Claim 6, further comprising a flow connector adapted for mounting on said cartridge (266), said flow connector (288) including a base (290) and wherein the penetrating means comprises two spaced hollow cannulas (296, 298), each pointed at each end, mounted within said base (290) in parallel relationship, the flow connector being movable relative to the cartridge from a first position in which said hollow cannulas are spaced from said chamber (272), to a second position in which said first and second cannulas have pierced said closure means (270), the second cannula (298) piercing the closure means (270) in alignment with said hollow open-ended member (280) such that the second cannula is disposed within said member, and the first cannula (296) piercing the closure means (270) at a point on said inner face (276) external to said member.

8. The administration set as in Claim 7, said base (290) including a chamber distant side (292), wherein said second cannula (298) extends from said chamber distant side of said base farther than said first cannula (296).

## Patentansprüche

1. Patrone (266) zum Einleiten eines gesundheitsfördernden Mittels in eine Fluidleitung, wobei die Patrone eine Wand aufweist, die eine langgestreckte Kammer (268) definiert, die ein gesundheitsförderndes Mittel (282) und eine durchstechbare, ein Ende der Kammer verschließende Verschlußeinrichtung (270) enthält, wobei die Verschlußeinrichtung (270) eine Außenfläche (276) und eine entgegengesetzte Innenfläche (278) hat, die der Kammer zugewandt ist, wobei die Verschlußeinrichtung von den Durchdringungseinrichtungen durchdringbar ist, um einen Fluiddurchfluß in die Kammer und aus der Kammer zu schaffen, dadurch gekennzeichnet, daß die Verschlußeinrichtung (270) mit einem hohlen offenendigen Element (280) versehen ist, das sich von der Innenfläche (278) in die Kammer in einer zu der Länge der Kammer im wesentlichen parallelen Richtung erstreckt, daß das gesundheitsfördernde Mittel in dem hohlen offenendigen Element (280) enthalten ist und daß die Verschlußeinrichtung (270) durchdringbar ist sowohl an einer ersten Stelle, um Fluiddurchfluß durch die Verschlußeinrichtung in das Element (280) zuzulassen, als auch an einer von dem Element beabstandeten zweiten Stelle, um Fluiddurchfluß aus der Kammer (268) zuzulassen.

2. Patrone nach Anspruch 1, wobei das gesundheitsfördernde Mittel (282) von einer flüssigkeitsdurchlässigen Barriere (284) in dem hohlen offenendigen Element (280) zurückgehalten wird.

3. Patrone nach Anspruch 2, wobei die Barriere (284) eine Barriere für teilchenförmige Stoffe aufweist.

4. Patrone nach Anspruch 3, wobei die Barriere (284) eine Nennporengröße von nicht mehr als 20 µm hat.

5. Patrone nach Anspruch 4, wobei die Barriere (284) eine Nennporengröße von nicht mehr 10 µm hat.

6. Parenterales Verabreichungsset, das eine Patrone (266) nach einem der vorhergehenden Ansprüche aufweist, wobei das Set ferner aufweist: ein Einlaßende (30), ein Auslaßende (34) und eine Fluiddurchflußbahn zwischen diesen Enden, wobei die Bahn einen Bereich aufweist, der mit Durchdringungseinrichtungen (296, 298) versehen ist, die einen Fluideinlaß (296b) in Kommunikation mit dem Einlaßende (30) und einen Fluidauslaß (298b) in Kommunikation mit dem Auslaßende (34) aufweisen, wobei die Verschlußeinrichtung (270) von den Durchdringungseinrichtungen durchdringbar ist, um den Fluideinlaß (296b) mit dem hohlen offenendigen Element (280) und den Fluidauslaß (298b) mit der Kammer (272) angrenzend an das hohle offenendige Element (280) in Verbindung zu bringen.

7. Verabreichungsset nach Anspruch 6, das ferner aufweist: einen Durchflußverbinder, der ausgebildet ist, um an der Patrone (266) angebracht zu werden, wobei der Durchflußverbinder (288) eine Basis (290) aufweist und wobei die Durchdringungseinrichtungen zwei beabstandete hohle Kanülen (296, 298) aufweisen, die jeweils an jedem Ende spitz und in der Basis (290) parallel zueinander angebracht sind, wobei der Durchflußverbinder relativ zu der Patrone aus einer ersten Position, in der die hohlen Kanülen von der Kammer (272) beabstandet sind, in eine zweite Position bewegbar ist, in der die erste und die zweite Kanüle die verschlußeinrichtung (270) durchstochen haben, wobei die zweite Kanüle (298) die Verschlußeinrichtung (270) in Ausfluchtung mit dem hohlen offenendigen Element (280) derart durchsticht, daß die zweite Kanüle in dem Element angeordnet ist, und die erste Kanüle (296) die Verschlußeinrichtung (270) an einem Punkt an der Innenfläche (276) außerhalb des Elements durchsticht.

8. Verabreichungsset nach Anspruch 7, wobei die Basis (290) eine von der Kammer ferne Seite (292) aufweist, wobei die zweite Kanüle (298) sich von der von der Kammer fernen Seite der Basis weiter als die erste Kanüle (296) erstreckt.

## Revendications

1. Cartouche (266), pour l'introduction d'un agent thérapeutique dans un conduit de fluide, comprenant une paroi qui définit une chambre allongée (268) contenant un agent thérapeutique (282) et un élément de fermeture perçable (270) fermant une extrémité de la chambre, l'élément de fermeture (270) présentant une face extérieure (276) et une face intérieure opposée (278) en regard de la chambre, l'élément de fermeture étant pénétrable par les moyens de pénétration pour créer un écoulement de fluide vers la chambre et hors de la chambre, caractérisée en ce que l'élément de fermeture (270) comporte un élément creux à extrémité ouverte (280) qui s'étend à partir de ladite face intérieure (278) et pénètre dans la chambre dans une direction sensiblement parallèle à la longueur de la chambre, en ce que l'agent thérapeutique est contenu dans l'élément creux à extrémité ouverte (280), et en ce que l'élément de fermeture (270) est pénétrable à la fois à un premier endroit, pour permettre l'entrée du fluide dans l'élément (280) à travers l'élément de fermeture, et à un deuxième endroit espacé de l'élément creux pour permettre au fluide de sortir de la chambre (268).

2. Cartouche suivant la revendication 1,
dans laquelle l'agent thérapeutique (282) est retenu dans l'élément creux à extrémité ouverte (280) au moyen d'une barrière perméable aux liquides (284).

3. Cartouche suivant la revendication 2,
dans laquelle ladite barrière (284) est une barrière d'arrêt de particules.

4. Cartouche suivant la revendication 3,
dans laquelle ladite barrière (284) a une dimension nominale de pore non supérieure à 20µm .

5. Cartouche suivant la revendication 4,
dans laquelle ladite barrière (224) a une dimension nominale de pore non supérieure à 10µm.

6. Ensemble d'administration parentérale comprenant une cartouche (266) suivant une quelconque des revendications précédentes, l'ensemble comprenant également une extrémité d'entrée (30), une extrémité de sortie (34) et un chemin d'écoulement de fluide entre lesdites extrémités, le chemin comportant une partie pourvue de moyens de pénétration (296,298) et comportant une entrée de fluide (296b) en communication avec ladite extrémité d'entrée (30) et une sortie de fluide (298b) en communication avec ladite extrémité de sortie (34), l'élément de fermeture (270) étant pénétrable par les moyens de pénétration pour placer l'entrée de fluide (296b) en communication avec ledit élément creux à extrémité ouverte (280) et la sortie de fluide (298b) en communication avec la chambre (272) près de l'élément creux à extrémité ouverte (280).

7. Ensemble d'administration suivant la revendication 6, comprenant en outre un connecteur d'écoulement prévu pour un montage sur ladite cartouche (266), ledit connecteur d'écoulement (288) comportant une base (290), et dans lequel les moyens de pénétration comprennent deux canules creuses espacées (296,298), ayant chacune une pointe à chaque extrémité, montées en parallèle dans ladite base (290), le connecteur d'écoulement étant déplaçable, par rapport à la cartouche, d'une première position dans laquelle lesdites canules creuses sont espacées de ladite chambre (272) à une deuxième position dans laquelle les dites première et deuxième canules ont percé ledit élément de fermeture (270), la deuxième canule (298) perçant l'élément de fermeture (270) en alignement avec ledit élément creux à extrémité ouverte (280) de sorte que la deuxième canule se trouve à l'intérieur dudit élément creux, et la première canule (296) perçant l'élément de fermeture (270) en un point de ladite face intérieure (276) située à l'extérieur du dit élément creux.

8. Ensemble d'administration suivant la revendication 7, ladite base (290) présentant une face (292) à l'opposé de la chambre, dans lequel ladite deuxième canule (298) s'étend, à partir de ladite face de ladite base à l'opposé de la chambre, plus loin que ladite première canule (296).
